(19) Europäisches Patentamt
European Patent Office
Office européen des brevets

(11) EP 2 868 656 A1

(12) **EUROPEAN PATENT APPLICATION**

(43) Date of publication:
**06.05.2015 Bulletin 2015/19**

(21) Application number: **13191659.5**

(22) Date of filing: **05.11.2013**

(51) Int Cl.:
***C07D 223/16** (2006.01)* ***A61K 31/55** (2006.01)*
***A61P 3/04** (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **LEK Pharmaceuticals d.d.
1526 Ljubljana (SI)**

(72) Inventor: **The designation of the inventor has not yet been filed**

(74) Representative: **Kluschanzoff, Harald
Sandoz International GmbH
Global Intellectual Property
Industriestrasse 25
83607 Holzkirchen (DE)**

(54) **Stabilized amorphous lorcaserin hydrochloride**

(57) The present invention provides stabilized amorphous lorcaserin hydrochloride ((*R*)-8-chloro-1-methyl-2,3,4,5-tetrahydro-1H-benzo[d]-azepine hydrochloride), a pharmaceutical composition comprising the same, as well as a process for obtaining the same.

EP 2 868 656 A1

## Description

The present invention is directed to stabilized amorphous lorcaserin hydrochloride, a pharmaceutical composition comprising the same, as well as a process for obtaining the same.

Obesity is a life-threatening disorder associated with an increased risk of mortality arising from concomitant diseases such as type II diabetes, hypertension, stroke, cancer and gallbladder disease.

WO 03/086306 A1 relates to $5HT_{2C}$ receptor modulators and discloses selective $5HT_{2C}$ agonists for reducing appetite and food consumption, thereby causing body weight loss. Lorcaserin ((R)-8-chloro-1-methyl-2,3,4,5-tetrahydro-1H-benzo[d]azepine) has been identified to be useful for the treatment of obesity.

Preparation and characterization of, amongst others salts, lorcaserin ((R)-8-chloro-1-methyl-2,3,4,5-tetrahydro-1H-benzo[d]azepine) hydrochloride is described in WO2005/019179 A2.

WO 2006/069363 A2 refers to crystalline forms of lorcaserin, namely three crystalline forms of lorcaserin hydrochloride, individually designated as Form I, Form II and Form III. Forms I and II are anhydrous, hygroscopic forms, both of which readily convert to Form III, a hemihydrate, upon exposure to moisture.

WO 2011/153206 A1 discloses a crystalline form of lorcaserin hydrochloride (Form IV), described to be a new anhydrous form, and its conversion to Form III, the lorcaserin hemihydrate, upon exposure to moisture.

A pharmaceutical composition has to be stable in an environment having enhanced relative humidity in order to be suitable for tropical countries. Forms I, II and IV (WO 2011/153206 A1) are hygroscopic and, thus, are problematic for the purpose of producing a pharmaceutical composition suitable for use in tropical countries. This is because a pharmaceutical composition comprising these solid crystalline forms of lorcaserin hydrochloride can be expected to absorb water when being exposed to an environment having a higher relative humidity.

However, also Form III of lorcaserin hydrochloride has a drawback that it can convert (at least partially) to another form, such as Form II, under particular environmental conditions, for example when exposed to elevated temperature (see WO 2011/153206 A1). Thus, medicaments comprising Form III may not be stable at particular environmental conditions, such as elevated temperature.

WO 2011/153206 A1 also refers to processes for the preparation of selective 5HT 2C agonists. Example 5 of said reference is directed to the thermodynamic relationships between lorcaserin hydrochloride salt hemihydrate Form III and the anhydrous polymorphs of lorcaserin hydrochloride salt. It is also described that at a critical water activity $a_w$ a given anhydrous form and lorcaserin hydrochloride salt hemihydrate will have equal solubility and can coexist in equilibrium. Below the critical $a_w$, the anhydrous form is described to be more thermodynamically stable, thus less soluble. Above the critical water activity, lorcaserin hydrochloride salt hemihydrate Form III is described to be more thermodynamically stable, thus less soluble.

The described water activity dependent equilibrium of lorcaserin hydrochloride hemihydrate Form III and the anhydrous polymorphs of lorcaserin hydrochloride salt can be a drawback for manufacturing a pharmaceutical composition based on lorcaserin hydrochloride Form III. This is because the pharmaceutical compositions comprising the Form III might not be obtained reliably or might not be stable upon storage with regard to the polymorphic form of the comprised lorcaserin hydrochloride. For example, the known crystalline forms of lorcaserin hydrochloride including Form III can be prone to polymorphic conversion when being exposed to an environment having an extreme relative humidity (high or low) and/or an elevated temperature.

For the above reasons, the known solid forms of lorcaserin hydrochloride need to be improved with respect to the physicochemical as well as the pharmaceutical characteristics of pharmaceutical compositions containing lorcaserin. Especially, the processability of the active pharmaceutical ingredient (API) lorcaserin hydrochloride during manufacture of the pharmaceutical composition and the characteristics of the finished dosage form, such as storage stability under difficult environmental conditions, e.g. extreme relative humidity and/or high temperature, need to be improved.

During the research of the present inventors aiming at improved dosage forms of lorcaserin hydrochloride, it has been discovered that it is not possible to obtain pure amorphous lorcaserin hydrochloride by standard methods. Without wishing to be bound to any theory, amorphous lorcaserin hydrochloride seems to apparently convert very quickly to one of the crystalline forms.

For example, lorcaserin hydrochloride was prepared from lorcaserin free base and a solution of hydrogen chloride according to the literature (Comparative Example 1). Fast solvent evaporation in a rotary evaporator yielded lorcaserin hydrochloride as a crystalline powder. A representative diffractogram is displayed in Figure 1.

Also in further attempts to prepare amorphous lorcaserin hydrochloride, such as (a) dissolving crystalline lorcaserin hydrochloride in water and ethanol, fast freezing the homogeneous solution in a liquid nitrogen bath and lyophilizing (Comparative Example 2), (b) putting crystalline lorcaserin hydrochloride (Form II) on a glass plate, heating over a Kofler bench until melting, followed by fast cooling over a metal plate at 0 °C and (c) by grinding experiments where crystalline lorcaserin hydrochloride, Form II or Form III, was manually grinded in a mortar, always lorcaserin hydrochloride showing the characteristic XRPD peaks of either Form III and/or Form II (according to WO 2011/153206 A1) has been obtained.

The various different attempts failing to prepare amorphous lorcaserin hydrochloride suggest that pure amorphous lorcaserin hydrochloride is not stable in the amorphous state and readily crystallizes.

The present invention therefore aims at a stable amorphous dosage form of lorcaserin hydrochloride avoiding the drawbacks of the known crystalline forms. It is thus the object of the present invention to develop new solid forms of lorcaserin hydrochloride used as an API in order to improve the performance profile of a pharmaceutical composition comprising said API.

**Summary of the invention**

In order to solve the technical problems related to the known dosage forms of lorcaserin hydrochloride, the present invention provides the following items.

1. Amorphous lorcaserin hydrochloride ((R)-8-chloro-1-methyl-2,3,4,5-tetrahydro-1H-benzo[d]azepine hydrochloride) of formula 1

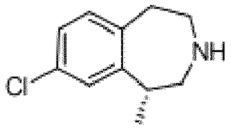

HCl

(formula 1)

stabilized by at least one pharmaceutically acceptable inorganic adsorbent.

2. Amorphous lorcaserin hydrochloride according to item 1, wherein the at least one pharmaceutically acceptable inorganic adsorbent is selected from MgO and $SiO_2$.

3. Amorphous lorcaserin hydrochloride according to item 1 or 2, wherein the at least one pharmaceutically acceptable inorganic adsorbent is a particulate having a surface area of at least 150 $m^2/g$, preferably of at least 200 $m^2/g$.

4. Amorphous lorcaserin hydrochloride according to any of item 1 to 3, wherein the at least one pharmaceutically acceptable inorganic adsorbent is $SiO_2$ selected from silica gel and fumed silica.

5. Amorphous lorcaserin hydrochloride according to any of items 1 to 4, wherein the silica gel and fumed silica is selected from Syloid® and Aerosil®.

6. Amorphous lorcaserin hydrochloride according to any of items 1 to 5, wherein the Syloid® and Aerosil® are selected from Syloid® 72 FP, Syloid® 244 FP, Aerosil® 200, Aerosil® 300 and Aerosil® 380.

7. Amorphous lorcaserin hydrochloride according to any of items 1 to 3, wherein the at least one pharmaceutically acceptable inorganic adsorbent is MgO selected from NanoActive® MgO.

8. Amorphous lorcaserin hydrochloride according to any of items 1 to 7, wherein the loading of amorphous lorcaserin hydrochloride on the adsorbent is 12% or more.

9. Amorphous lorcaserin hydrochloride according to any of items 1 to 7, wherein the loading of amorphous lorcaserin hydrochloride on the adsorbent is 20% or more.

10. Amorphous lorcaserin hydrochloride according to any of items 1 to 7, wherein the loading of amorphous lorcaserin hydrochloride on the adsorbent is 30% or more.

11. Amorphous lorcaserin hydrochloride according to any of items 1 to 7, wherein the loading of amorphous lorcaserin hydrochloride on the adsorbent is 40% or more.

12. Amorphous lorcaserin hydrochloride according to any of items 1 to 11, wherein the loading of amorphous lorcaserin hydrochloride on the adsorbent is 65% or less.

13. Amorphous lorcaserin hydrochloride according to any of items 1 to 11, wherein the loading of amorphous

lorcaserin hydrochloride on the adsorbent is 60% or less.

14. Amorphous lorcaserin hydrochloride according to any of items 1 to 11, wherein the loading of amorphous lorcaserin hydrochloride on the adsorbent is 55% or less.

15. Process for the preparation of amorphous lorcaserin hydrochloride stabilized by at least one pharmaceutically acceptable inorganic adsorbent, the method comprising the steps (i) to (iv):

(i) dissolving lorcaserin hydrochloride in a protic solvent, an aprotic polar solvent, or a mixture thereof, or dissolving lorcaserin in said protic solvent, aprotic polar solvent, or mixture thereof upon *in situ* generation of lorcaserin hydrochloride;
(ii) adding at least one pharmaceutically acceptable inorganic adsorbent to the solution obtained in step (i);
(iii) mixing the dispersion obtained in step (ii); and
(iv) removing the solvent;

wherein the at least one pharmaceutically acceptable inorganic adsorbent may alternatively be dispersed in the protic solvent, aprotic polar solvent, or mixture thereof, before dissolving the lorcaserin or lorcaserin hydrochloride.

16. Process according to item 15, wherein the least one pharmaceutically acceptable inorganic adsorbent is dispersed in step (ii) after dissolving lorcaserin or lorcaserin hydrochloride in step (i).

17. Process according to item 15 or 16, wherein the protic solvent in step (i) is a C1 to C4 alcohol, preferably methanol, ethanol or 2-propanol.

18. Process according to any of items 15 to 17, wherein the aprotic polar solvent in step (i) is a halogenated C1 to C4 hydrocarbon, preferably dichloromethane, or acetonitrile.

19. Process according to any of items 15 to 18, wherein the step (i) is carried out at a temperature of 10 to 60 °C, preferably at 18 to 30 °C, more preferably 20 to 25 °C (r.t.), and further wherein when the solvent in the step (i) is 2-propanol or acetonitrile, a preferred temperature is 40 to 60 °C.

20. Process according to any of items 15 to 19, wherein the at least one pharmaceutically acceptable inorganic adsorbent is selected from MgO and $SiO_2$.

21. Process according to any of items 15 to 20, wherein the at least one pharmaceutically acceptable inorganic adsorbent is a particulate having a surface area of at least 150 $m^2/g$, preferably of at least 200 $m^2/g$.

22. Process according to any of items 15 to 21, wherein the at least one pharmaceutically acceptable inorganic adsorbent is $SiO_2$ selected from silica gel and fumed silica.

23. Process according to any of items 15 to 22, wherein the silica gel and fumed silica is selected from Syloid® and Aerosil®.

24. Process according to any of items 15 to 23, wherein the Syloid® and Aerosil® are selected from Syloid® 72 FP, Syloid® 244 FP, Aerosil® 200, Aerosil® 300 and Aerosil® 380.

25. Process according to any of items 15 to 21, wherein the at least one pharmaceutically acceptable inorganic adsorbent is MgO selected from NanoActive® MgO.

26. Process according to any of items 15 to 25, wherein the solvent is removed in step (iv) by filtration.

27. Process according to any of items 15 to 25, wherein the solvent is removed in step (iv) by evaporation, preferably under reduced pressure.

28. A pharmaceutical composition comprising stabilized amorphous lorcaserin hydrochloride ((R)-8-chloro-1-methyl-2,3,4,5-tetrahydro-1H-benzo[d]azepine hydrochloride) according to any of items 1 to 14.

29. The pharmaceutical composition according to item 28, which further comprises at least one pharmaceutically

acceptable ingredient.

30. The pharmaceutical composition according to item 28 or 29 for use as a medicament.

31. The pharmaceutical composition according to item 30 for use in the treatment of obesity.

32. The pharmaceutical composition according to any of items 28 to 31, which is formulated for oral application.

33. The pharmaceutical composition according to any of items 28 to 32, which is in the form of tablets, capsules, pills or lozenges.

34. The pharmaceutical composition according to any of items 28 to 33, which comprises 5 to 100 mg, preferably 7 to 20 mg, amorphous lorcaserin hydrochloride per dosage form.

35. The pharmaceutical composition according to any of items 28 to 34, which is a modified release dosage form.

**Detailed Description**

Hereinafter, the present invention will be described in more detail supplementing the above items. For the purposes of interpreting the applied terms, all terms as used herein are to be interpreted in accordance with their everyday meaning to the person of ordinary skill in the art unless an explicit definition is given hereinafter.

In order to circumvent the problems associated with the known crystalline forms of lorcaserin hydrochloride, the present invention provides amorphous lorcaserin hydrochloride ((*R*)-8-chloro-1-methyl-2,3,4,5-tetrahydro-1H-benzo[d]azepine hydrochloride) of formula 1

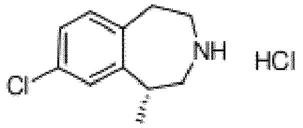

(formula 1)

stabilized by at least one pharmaceutically acceptable inorganic adsorbent.

As used herein, the term "amorphous lorcaserin hydrochloride" is intended to mean a non-crystalline form of lorcaserin hydrochloride as determined by differential scanning calorimetry (DSC), X-ray powder diffraction (XRPD) analysis or by using a scanning electron microscope (SEM). Especially the absence of peaks on the X-ray powder diffractogram of the stabilized amorphous lorcaserin hydrochloride of the present invention, in particularly the absence of characteristic XRPD peaks for Form III of lorcaserin hydrochloride, suggests that the lorcaserin hydrochloride is amorphous. Characteristic X-ray powder diffraction pattern of Form I comprises peaks at 2-theta angles of 11.5 ± 0.2°, 14.5 ± 0.2°, 16.0 ± 0.2°, 23.0 ± 0.2° and 23.3 ± 0.2°. Characteristic X-ray powder diffraction pattern of Form II comprises peaks at 2-theta angles of 6.5 ± 0.2°, 12.9 ± 0.2°, 17.1 ± 0.2°, 17.5 ± 0.2° and 18.5 ± 0.2°. Characteristic X-ray powder diffraction pattern of Form III comprises peaks at 2-theta angles of 13.7 ± 0.2°, 14.9 ± 0.2°, 15.4 ± 0.2°, 16.7 ± 0.2° and 19.2 ± 0.2°. Characteristic X-ray powder diffraction pattern of Form IV comprises peaks at 2-theta angles of 8.9 ± 0.2°, 10.7 ± 0.2°, 15.3 ± 0.2°, 17.8 ± 0.2° and 18.3 ± 0.2°.

According to the research of the present inventors, it is possible to provide stable amorphous lorcaserin hydrochloride by stabilizing it with an inorganic adsorbent. As used herein, the term "inorganic adsorbent" is intended to mean a solid supporter represented by any inorganic substance onto which amorphous lorcaserin hydrochloride can be adsorbed and which is pharmaceutically acceptable. The adsorption of amorphous lorcaserin hydrochloride onto the inorganic adsorbent serves to stabilize the lorcaserin hydrochloride in its amorphous form. In this way, the lorcaserin hydrochloride is not simply deposited on the adsorbent, but stabilized by non-covalent forces as can be confirmed by methods known in the art, such as scanning electron microscopy (SEM) visualization or infrared (IR) shift. In order to excel the stabilizing effect efficiently, the inorganic adsorbent should preferably have a certain surface area and/or porosity, such that the lorcaserin hydrochloride can be adsorbed on the outer and/or inner surface of the adsorbent. The surface area of the inorganic adsorbent usually has a BET-surface area of at least 1 $m^2/g$, preferably of at least 50 $m^2/g$, more preferably of at least 100 $m^2/g$, still more preferably of at least 150 $m^2/g$, and most preferably of at least 200 $m^2/g$. The upper limit of the surface area is not essentially limited, but may usually be 10000 $m^2/g$ or less, preferably 5000 $m^2/g$ or less, still more preferably 1000 $m^2/g$ or less, most preferably 750 $m^2/g$ or less. The upper and lower limits are fully combinable and a suitable surface area may preferably range 100 to 5000 $m^2/g$, more preferably 150 to 1000 $m^2/g$, still

more preferably of from 200 to 750 $m^2$/g. The determination of the BET-surface area of the carrier can be carried out according to the method as described in the article: *J. Am. Chem. Soc.* 60, 309 (1938). Additionally, the inorganic adsorbent with the defined BET-surfaces has preferably a certain degree of porosity (open porosity determined according to DIN EN 623-2) of at least 10 %, 20 %, 30 %, 40 %, or 60 %. Suitably, the porosity ranges from 10 to 70 %, preferably from 20 to 70 %, more preferably from 30 to 70 % and still more preferably from 40 to 70%. The size of the inorganic adsorbent, preferably in the particulate form is not specifically limited and may suitable be in the micro or nano scale. A particulate inorganic adsorbent may be present as single particles or agglomerate thereof, preferably having an average particle size (via Malvern) of 20 $\mu$m or less, more preferably 15 $\mu$m or less and preferably of at least 5 nm, preferably of at least 10 nm.

Non-limiting preferred examples of the inorganic adsorbents include $SiO_2$ and MgO. Preferably, the inorganic adsorbent is silicon dioxide selected from silica gel and fumed silica, i.e. synthetic and amorphous silicas. Such silicas may more preferably be selected from Aerosil, for example Aerosil 200, Aerosil 300, and Aerosil 380 (Evonik Industries), and Syloid, for example Syloid AL1, Syloid 72 FP and Syloid 244 FP (W. R. Grace & Co.-Conn.). If choosing magnesium oxide as the inorganic adsorbent, an especially preferred MgO is represented by NanoActive® MgO featuring a high porosity and large BET surface area.

By means of providing the above described inorganic adsorbent, the present invention is able to achieve high loadings of the lorcaserin hydrochloride. As a result, it is possible to achieve a stable form of amorphous lorcaserin hydrochloride stabilized by small amounts of the inorganic adsorbent. Consequently, the presence of the adsorbent does not have much influence on the volume of the final dosage form of the amorphous lorcaserin hydrochloride.

As used herein, the percentage loadings of amorphous lorcaserin hydrochloride on the adsorbent are calculated as follows:

loading (%) = ((mass of the obtained dry adsorbate) - (mass of inorganic adsorbent weighed at the beginning)/ (mass of the obtained dry adsorbate)) × 100.

Loadings can also be measured by spectrometric assay methods.

Although the lower limit of the loading of the inorganic adsorbent with lorcaserin hydrochloride is not specifically limited, it is generally desirable to achieve a loading as high as possible in order to minimize the influence of inorganic adsorbent with respect to the content/volume in the final dosage form. Preferably, the loading of lorcaserin hydrochloride is at least 12 %, more preferably at least 20 %, still more preferably at least 30 %, and most preferred at least 40 %. Although the upper limit of the loading of lorcaserin hydrochloride is not specifically limited and it is generally desirable to achieve a loading as high as possible, it is also desirable that essentially all of lorcaserin hydrochloride is present in amorphous from adsorbed to the inorganic adsorbent. If the loading percentage becomes too high, it may in the individual case be difficult to realize a condition of lorcaserin hydrochloride being entirely adsorbed to the inorganic adsorbent in amorphous state. Therefore, it is generally preferred that the upper limit of the loading is 65 % or less, more preferably 60 % or less, still more preferably 55 % or less.

The above described stabilized amorphous lorcaserin hydrochloride of the present invention may be obtained by a method comprising the steps (i) to (iv):

(i) dissolving lorcaserin hydrochloride in a protic solvent, an aprotic polar solvent, or a mixture thereof, or dissolving lorcaserin in said protic solvent, aprotic polar solvent, or mixture thereof upon *in situ* generation of lorcaserin hydrochloride;
(ii) adding at least one pharmaceutically acceptable inorganic adsorbent to the solution obtained in step (i);
(iii) mixing the dispersion obtained in step (ii); and
(iv) removing the solvent.

Although it is also possible that the at least one pharmaceutically acceptable inorganic adsorbent is alternatively initially dispersed in the protic solvent, aprotic polar solvent, or mixture thereof, before dissolving the lorcaserin or lorcaserin hydrochloride in the resulting dispersion, it is preferred that the at least one pharmaceutically acceptable inorganic adsorbent is dispersed in step (ii) after dissolving lorcaserin or lorcaserin hydrochloride in step (i).

The protic solvent, aprotic polar solvent or mixture thereof is not specifically limited as long as it is a solvent or mixture of solvents that is able to dissolve the lorcaserin hydrochloride while not dissolving the inorganic adsorbent. As used herein, the term "aprotic solvent" is intended to mean any solvent which cannot readily donate hydrogen atoms as protons ($H^+$), i.e. are not acidic. Non-limiting examples of aprotic solvents include $CH_2Cl_2$. As used herein, the term "protic solvent" is intended to mean any solvent which contains one or more labile hydrogen atoms that are capable of

hydrogen bonding and which can be readily donated as protons ($H^+$). Non-limiting examples of protic solvents include alcohols.

Due to the favorable attributes to be able to dissolve lorcaserin hydrochloride in high concentrations while not dissolving the inorganic adsorbent, which is preferably selected from the above described silicas and/or MgO, the protic solvent used in step (i) is suitably a C1 to C4 alcohol, most preferably methanol, ethanol or 2-propanol. The aprotic polar solvent used in step (i) is preferably a halogenated C1 to C4 hydrocarbon, such as dichloromethane, 1,2-dichloroethane, or acetonitrile, more preferably dichloromethane. In view of a more environmental-friendly and economic process under less hazardous/toxic conditions, it is preferred to take advantage of an alcoholic medium, preferably methanol, ethanol or 2-propanol.

The conditions for the step (i) are not essentially limited and the step (i) may proceed at any suitable temperature. It is preferred that the temperature is below the boiling point of the solvent or solvent mixture. Under environmental and economical aspects, it is preferred to apply a temperature in the range of 10 to 60°C, preferably 18 to 30°C, most preferably at room temperature (r.t.; 20 to 25°C), if 2-propanol or acetonitrile are used as solvents the preferred temperatures are higher, e.g., 40-60°C). In case the lorcaserin is dissolved as a free base, the free base is preferably converted to the hydrochloride salt by adding a solution providing hydrogen chloride.

Further, the inorganic adsorbent added in the step (ii) may be the same as described above. Preferably, the inorganic adsorbent is selected from the above described fumed silica or silica gels selected from the Aerosil® or Syloid® series or porous MgO having a large surface area, preferably NanoActive® MgO.

The step (iii) is not specifically limited and the mixing may be accomplished by conventional mixing devices, such as a stirrer, an ultrasonic bath etc. The mixing time is not specifically limited and may suitable be adjusted taking into consideration the solvent, the concentration of lorcaserin hydrochloride and the kind and amount of the inorganic adsorbent. The mixing time is preferably adjusted to ensure a uniform distribution of the lorcaserin hydrochloride on the inorganic adsorbent. Usually, the mixing proceeds within a few minutes. The upper limit for the mixing time is not specifically limited. However, additional mixing time is not required in case the additional time does not contribute to improved uniformity any more. Usually, a mixing time of 1 hour is sufficient to attain satisfactory results, while a mixing time is preferably not more than 45 minutes. Preferably the mixing time is in a range between 5 to 45 minutes, more preferably 10 to 40 minutes.

The solvent can be removed in the step (iv) using any known method. Preferably the solvent is removed by filtration or evaporation or by a combination of evaporation and filtration.

In case not all of the lorcaserin hydrochloride dissolved in the solvent is adsorbed to the inorganic adsorbent, the excessive amount thereof will remain in the filtrate after removing the solvent by filtration. Such lorcaserin hydrochloride contained in the filtrate may preferably be recovered for subsequent loading of inorganic adsorbents.

Preferably, the solvent is removed in the step (iv) by evaporation. This is because the evaporation method enables to attain a higher loading of the inorganic adsorbent by lorcaserin hydrochloride. In order to achieve a high loading of lorcaserin hydrochloride being fully adsorbed to the inorganic adsorbate in an amorphous state, it is preferred to carry out the evaporation in such a manner that the solvent or solvent mixture is evaporated slowly, suitably during a period (evaporation period) of at least 10 minutes, preferably at least 30 minutes, further preferred at least 50 minutes, further preferred at least 70 minutes, further preferred at least 100 minutes, further preferred at least 120 minutes, more preferably during a period of at least 2.5 hours. Within the context of the present invention, the "evaporation period" corresponds to the time that is required to evaporate at least 80%, further preferred at least 90%, and further preferred at least 95% of the solvent. A too fast evaporation of the solvent may lead to the formation of e.g. coprecipitates of lorcaserin hydrochloride, which would not be adsorbed on the carrier and which would be in the form of amorphous or crystalline particles. Slowly removing the solvent has the benefit that uneconomic, complex and laborious process steps are not necessary, which would otherwise be necessary to achieve a fast removal. Additionally, the slow removal of the solvent leads to the formation of a stable adsorbate having improved properties with respect to stability and solubility of lorcaserin hydrochloride.

The amorphous lorcaserin hydrochloride stabilized by the at least one inorganic adsorbent is preferably manufactured in a solid dry state. If solvent remains in the adsorbate after the filtration and/or evaporation in step (iv), the residual solvent is preferably removed to pharmaceutically acceptable levels by a drying step carried out by any conventional drying means. If carrying out a drying step, the step is preferably carried out under mild conditions, more preferably under reduced pressure. Preferably, the evaporation in the step (iv) is carried out in a manner leading to a solid dry product without the need for an additional drying step.

Furthermore, the present invention relates to a pharmaceutical composition comprising the above described stabilized lorcaserin hydrochloride. The present invention also relates to the use of the pharmaceutical composition as a medicament and especially to the use for the treatment of obesity. The term "pharmaceutical composition" is intended to mean any mixture or solution containing amorphous lorcaserin hydrochloride stabilized by an inorganic adsorbent suitable for administration to a mammal, preferably a human, in order to prevent, treat or control a particular disease or condition affecting the mammal.

Among other advantages, the stabilized amorphous lorcaserin hydrochloride according to the present invention allows for the easier preparation of modified release dosage forms containing lorcaserin hydrochloride. In a modified release dosage form the lorcaserin hydrochloride can be released gradually over a relatively long period so that the drug is maintained in the blood stream for a long time and at a more uniform concentration as compared to an immediate release dosage form. A modified release dosage form comprising locaserin hydrochloride is desirable because it addresses known side effects of lorcaserin hydrochloride, such as a tendency to cause sedation in susceptible subjects.

Within the present invention, the term "modified release" is generally used as defined by the US Pharmacopeia (USP). Preferably, modified release dosage forms comprising the stabilized amorphous lorcaserin hydrochloride of the present invention are those whose drug release characteristics accomplish therapeutic or convenience objectives not offered by immediate release forms. Generally, immediate release (IR) forms release at least 70 percent of the drug within 1 hour or less. The term "modified release" can comprise delayed release, prolonged release, sustained release, extended release and/or controlled release. Delayed release usually indicates that the drug (i.e., lorcaserin hydrochloride) is not being released immediately after administration but at a later time, preferably less than 10 percent are released within two hours after administration. Prolonged release usually indicates that the drug (i.e., lorcaserin hydrochloride) is provided for absorption over a longer period of time than IR forms, preferably for about 2 to 24 hours, in particular for 3 to 12 hours. Sustained release usually indicates an initial release of drug (i.e., lorcaserin hydrochloride), sufficient to provide a therapeutic dose soon after administration, preferably within two hours after administration, and then a gradual release after an extended period of time, preferably for about 3 to 18 hours, in particular for 4 to 8 hours. Extended release usually indicates a slow drug (i.e., lorcaserin hydrochloride) release, so that plasma concentrations are maintained at a therapeutic level for a time period of between 6 and 36 hours, preferably between 8 and 24 hours. Controlled release dosage forms usually release the drug (i.e., lorcaserin hydrochloride) at a constant rate and provide plasma concentrations that remain essentially invariant with time. The preparation of the various modified release dosage forms starting from the solid dispersion of the present invention is well within the capabilities of the person skilled in the art, as exemplified by standard pharmaceutical textbooks such as the Encyclopedia of Pharmaceutical technology.

A preferred formulation of the pharmaceutical composition may be an oral dosage form and particularly preferred are solid formulations such as capsules, tablets, pills and lozenges.

The stabilized amorphous lorcaserin hydrochloride according to the present invention may be directly used as powders (micronized particles) or granules, or it may be combined together with one or more pharmaceutically acceptable ingredients in admixing the components and optionally finely divide them, and then filling capsules, composed for example from hard or soft gelatin, compressing tablets, pills or lozenges. Optionally, coatings may be applied after compression to form pills.

In a preferred embodiment, the pharmaceutical composition is a solid oral dosage form, in particular an extended release solid oral dosage form.

Pharmaceutically acceptable ingredients are well known for the various types of formulations and may be for example binders such as natural or synthetic polymers, excipients, disintegrants, lubricants, surfactants and/or co-surfactants, sweetening and other flavouring agents, coating materials, preservatives, dyes, thickeners, fillers, adjuvants, antimicrobial agents and carriers for the various formulation types. One of ordinary skill in the art may select one or more of the aforementioned ingredients with respect to the particular desired properties of the dosage form by routine experimentation and without any undue burden. The amount of each ingredient used may vary within ranges conventional in the art. The following references disclose techniques and ingredients used to formulate oral dosage forms (see The Handbook of Pharmaceutical Excipients, 4th edition, Rowe et al., Eds., American Pharmaceuticals Association (2003); and Remington: the Science and Practice of Pharmacy, 20th edition, Gennaro, Ed., Lippincott Williams & Wilkins (2000).

Examples for binders comprise gum tragacanth, acacia, starch, gelatin, and biological de-gradable polymers such as homo- or co-polyesters of dicarboxylic acids, alkylene glycols, polyalkylene glycols and/or aliphatic hydroxyl carboxylic acids; homo- or co-polyamides of dicarboxylic acids, alkylene diamines, and/or aliphatic amino carboxylic acids; corresponding polyester-polyamide-copolymers, polyanhydrides, polyortho-esters, polyphosphazene and polycarbonates. The biological degradable polymers may be linear, branched or crosslinked. Specific examples are polyglycolic acid, polylactic acid, and poly-d,l-lactide/glycolide. Other examples for polymers are water-soluble polymers such as polyoxaalkylenes (polyoxaethylene, polyoxapropylene and mixed polymers thereof), poly-acrylamides and hydroxylalkylated polyacrylamides, polymaleic acid and esters or -amides thereof, polyacrylic acid and esters or -amides thereof, polyvinylalcohol and esters or -ethers thereof, polyvinylimidazole, polyvinylpyrrolidone, and natural polymers like chitosan, carragenan or hyaluronic acid.

Suitable disintegrants which can be used for the pharmaceutical compositions of the present invention comprise e.g. starch, ion exchange resins, e.g. Amberlite, cross-linked polyvinylpyrrolidone, modified cellulose gum, e.g croscarmellose sodium, sodium starch glycolate, sodium carboxymethylcellulose, sodium dodecyl sulphate, modified corn starch, microcrystalline cellulose, magnesium aluminium silicate, alginic acid, alginate and powdered cellulose.

Suitable lubricants which can also be used for the pharmaceutical compositions of the present invention comprise e.g. magnesium stearate, calcium stearate, stearic acid, talc, polyethylene glycol, sodium lauryl sulphate and

magnesium lauryl sulphate.

Surfactants may be anionic, amphoteric or neutral. Examples for surfactants are lecithin, phospholipids, octyl sulfate, decyl sulfate, dodecyl sulfate, tetradecyl sulfate, hexadecyl sulfate and octadecyl sulfate, 1-acylamino-ethane-2-sulfonic acids, such as 1-octanoylaminoethane-2-sulfonic acid, 1-decanoyl-aminoethane-2-sulfonic acid, 1-dodecanoylaminoethane-2-sulfonic acid, 1-tetra-decanoylaminoethane-2-sulfonic acid, 1-hexadecanoylaminoethane-2-sulfonic acid, and 1-octadecanoylaminoethane-2-sulfonic acid, and taurocholic acid and taurodeoxycholic acid, bile acids and their salts, such as cholic acid, deoxycholic acid and sodium glycocholates, sodium caprate or sodium laurate, sodium oleate, sodium lauryl sulphate, sodium cetyl sulphate, sulfated castor oil and sodium dioctyl sulfosuccinate, cocamidopropylbetaine and laurylbetaine, fatty alcohols, cholesterols, glycerol mono- or distearate, glycerol mono- or -dioleate and glycerol mono- or -dipalmitate, and polyoxyethylene stearate.

Examples for coating materials are gelatin, wax, shellac or biological degradable polymers.

Examples for preservatives are methyl-, propyl-, or butylparabens, propylene paraben, sorbic acid, chlorobutanol, phenol, thimerosal, potassium sorbate, glycerin, propylene glycol, cysteine and/or methionine.

Examples for thickeners are synthetic polymers, fatty acids and fatty acid salts and esters and fatty alcohols.

Examples of fillers include confectioner's sugar, compressible sugar, dextrates, dextrin, dextrose, lactose, mannitol, microcrystalline cellulose, powdered cellulose, sorbitol, sucrose and talc.

The formulation according to the invention may also contain isotonic agents, such as sugars, buffers or sodium chloride.

Suitable dosage forms include solid dosage forms, like tablets, powders, capsules, suppositories, sachets, troches and lozenges.

Capsules typically contain a solid composition within a capsule, which may be made of gelatin or other conventional encapsulating materials. Tablets and powders may be coated, e.g. with an enteric coating. The enteric coated powder forms may have coatings comprising phthalic acid cellulose acetate, hydroxypropylmethylcellulose phthalate, polyvinylalcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric coating.

As described above, modified release formulations may also be prepared from the stabilized amorphous lorcaserin hydrochloride according to the invention in order to achieve a more controlled release of the API in contact with the body fluids in the gastro intestinal tract, and to provide a substantial constant and effective level of the API in the gastric juice. For this purpose, the stabilized amorphous lorcaserin hydrochloride of the present invention may be embedded in a polymer matrix of a biological degradable polymer, a water soluble polymer or a mixture of both, and optionally suitable surfactants. Embedding can mean in this context the incorporation of micro-particles in a matrix of polymers. Modified release formulations can also be obtained through encapsulation of dispersed micro-particles or emulsified micro-droplets via known dispersion or emulsion coating technologies.

The present invention further relates to a solid oral dosage form, such as a tablet, which may be prepared by wet granulation comprising the steps of:

(a) dry blending the stabilized amorphous solid lorcaserin hydrochloride of the present invention and a part of the diluent,
(b) preparing a binder solution by dissolving a binder and a wetting agent in a suitable solvent,
(c) spraying the binder solution of step (b) on the mixture obtained in step (a),
(d) drying the obtained granulate and sieving the same,
(e) mixing the obtained granulate with the remaining part of diluent and a disintegrant,
(f) adding an optional glidant and/or an optional lubricant to the mixture,
(g) compressing the obtained mixture into a tablet, and
(h) film-coating the obtained tablet.

Suitable solvents in step (b) of the herein disclosed wet granulation process are e.g. water, acetic acid, acetone, anisole, 1-butanol, 2-butanol, butyl acetate, tert-butylmethyl ether, cumene, dimethyl sulfoxide, ethanol, ethyl acetate, ethyl ether, ethyl formate, formic acid, heptane, isobutyl acetate, isopropyl acetate, methyl acetate, 3-methyl-1-butanol, methylethyl ketone, methylisobutyl ketone, 2-methyl-1-propanol, pentane, 1-pentanol, 1-propanol, 2-propanol, propyl acetate and tetrahydrofuran.

Formulations of the present invention typically comprise 5 to 100 mg, preferably 7 to 20 mg, more preferably about 10 mg of lorcaserin.

Other objects, features, advantages and aspects of the present invention will become apparent to those of skill from the following description. It should be understood, however, that the description and the following specific examples, while indicating preferred embodiments of the invention, are given by way of illustration only. Various changes and

modifications within the spirit and scope of the disclosed invention will become readily apparent to those skilled in the art from reading the description and the other parts of the present disclosure.

The present invention is illustrated in the following examples, which should not be construed as limiting.

## Examples

Analytical Methods:

X-Ray Powder Diffraction (XRPD)

X-Ray powder diffractograms (XRPD) were obtained with an X'Pert PRO diffractometer (PANalytical, Almelo, The Netherlands) equipped with a theta/theta coupled goniometer in transmission geometry, programmable XYZ stage with well plate holder, Cu-$K_{\alpha 1,2}$ radiation source (wavelength 0.15419 nm) and a solid state PIX'cel detector. The diffractograms were recorded at a tube voltage of 45 kV, a tube current of 40 mA applying a stepsize of 0.013° 2-theta with 40 s per step (255 channels) in the angular range of 2° to 40° 2-theta at ambient conditions. A typical precision of the 2-theta values is in the range of about $\pm$ 0.2° 2-theta. Thus, a diffraction peak that appears at 5.0° 2-theta can appear between 4.8 and 5.2° 2-theta on X-ray diffractometers under standard conditions.

Differential Scanning Calorimetry (DSC)

DSC thermograms were obtained using a Mettler Toledo DSC822e differential scanning calorimeter. The sample (1-10 mg) was placed in an unsealed aluminium pan with a hole and heated at 10°C/min in the temperature range from 30 °C to 250 °C.

Scanning Electron Microscopy

The samples is mounted on stubs with double faced adhesive tape and sputter coated with a 3 nm gold layer in a high vacuum sputter coater (Leica EM SCD 500). Surface topography is analyzed with a scanning electron microscope (JSM FE SEM 7001, Jeol, Tokyo, Japan) using secondary electron imaging (SEI) detector and different magnifications (100 - 20 000x). An acceleration voltage of 2 kV and a working distance of 5 mm is used.

**Example 1:** Adsorption of (*R*)-8-chloro-1-methyl-2,3,4,5-tetrahydro-1H-benzo[*d*]azepin-3-ium chloride on silica (Aerosil® 200) as solid supporter

Into a flask equipped with magnetic stir bar was placed (*R*)-8-chloro-1-methyl-2,3,4,5-tetrahydro-1H-benzo[*d*]azepin-3-ium chloride (400 mg) which was dissolved in dichloromethane (25 mL). Afterwards Aerosil® 200 (1 g) was added and the suspension was stirred at r.t. for 15 minutes. Solid was filtered off, washed with dichloromethane (2 x 5 mL) and dried under vacuum to obtain adsorbate (1.19 g; 16 % loading). Adsorbate was characterized with DSC and XPRD analysis where only amorphous material was detected within the detection limits. The filtrate was concentrated and the salt was recovered.

Example 2: Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (200 mg) which was dissolved in dichloromethane (20 mL). Syloid® 72 FP (1 g) was added and the suspension was stirred at r.t. for 15 minutes. Solid was filtered off, washed in dichloromethane (2 x 5 mL) and dried under vacuum to obtain adsorbate (1.16 g; 14 % loading). Adsorbate was characterized with DSC and XPRD analysis where only amorphous material was detected within detection limits. The filtrate was concentrated for recovery of the salt.

**Example 3:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (3.5 g) which was dissolved in dichloromethane (100 mL). Syloid® 72 FP (4.9 g) was added and the suspension was stirred at r.t. for 30 minutes. Solvent was than slowly evaporated under reduced pressure and dried under vacuum to obtain dry adsorbate (8.35 g; >40 % loading). Adsorbate was characterized with DSC and routine XPRD analysis where only amorphous material was detected within detection limits. Stress stability of obtained dry

material according to reference sample (lorcaserin hydrochloride crystalline form) was also tested and no sensitivity on temperature effect (60 °C), moisture effect (29-75% relative humidity) and oxidative stress (oxygen atmosphere) was detected. Assay of lorcaserin hydrochloride in adsorbate form did not change under stress conditions.

**Example 4:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was dissolved in dichloromethane (8 mL). Syloid® 72 FP (300 mg) was added in portions and the suspension was stirred at r.t. for 30 minutes. Solvent was than slowly evaporated under reduced pressure and dried under vacuum to obtain dry adsorbate (595 mg; 50 % loading). Adsorbate was characterized with DSC analysis where only amorphous material was detected within detection limits.

**Example 5:** Adsorption of (*R*)-8-chloro-1-methyl-2,3,4,5-tetrahydro-1H-benzo[*d*]azepin-3-ium chloride on silica (Syloid® 244 FP) as solid supporter

Into a flask equipped with magnetic stir bar was placed (*R*)-8-chloro-1-methyl-2,3,4,5-tetrahydro-1H-benzo[*d*]azepin-3-ium chloride (3.5 g) which was dissolved in dichloromethane (100 mL). Syloid® 244 FP (7 g) was added and the suspension was stirred at r.t. for 30 minutes. Solvent was than evaporated under reduced pressure and dried under vacuum to obtain dry adsorbate (10.45 g; 33 % loading). Adsorbate was characterized with DSC and routine XPRD analysis where only amorphous material was detected within detection limits. Stress stability of obtained dry material according to reference sample (lorcaserin hydrochloride crystalline form) was also tested and no sensitivity on temperature effect (60 °C), moisture effect (25-75% relative humidity) and oxidative stress (oxygen atmosphere) was detected. Assay of lorcaserin hydrochloride in adsorbate form did not change under stress conditions.

**Example 6:** Adsorption of (*R*)-8-chloro-1-methyl-2,3,4,5-tetrahydro-1H-benzo[*d*]azepin-3-ium chloride on silica (Syloid® 244 FP) as solid supporter

Into a flask equipped with magnetic stir bar was placed (*R*)-8-chloro-1-methyl-2,3,4,5-tetrahydro-1H-benzo[*d*]azepin-3-ium chloride (500 mg) which was dissolved in dichloromethane (10 mL). Syloid® 244 FP (700 mg) was added in portions and the suspension was stirred at r.t. for 30 minutes. Solvent was than slowly evaporated under reduced pressure and dried under vacuum to obtain dry adsorbate (1.14 g; >39 % loading). Adsorbate was characterized with DSC and XPRD analysis where amorphous material was detected within detection limits.

**Example 7:** Adsorption of (*R*)-8-chloro-1-methyl-2,3,4,5-tetrahydro-1H-benzo[*d*]azepin-3-ium chloride on magnesium oxide as solid supporter

Into a flask equipped with magnetic stir bar was placed (*R*)-8-chloro-1-methyl-2,3,4,5-tetrahydro-1H-benzo[*d*]azepin-3-ium chloride (1 g) which was dissolved in dichloromethane (10 mL). NanoActive® magnesium oxide (2 g) was added and the suspension was stirred at r.t. for 30 minutes. Solvent was than evaporated under reduced pressure and dried under vacuum to obtain dry adsorbate (2.95 g; >32 % loading). Adsorbate was characterized with DSC and XPRD analysis where only amorphous material was detected within detection limits.

**Example 8:** Adsorption of (*R*)-8-chloro-1-methyl-2,3,4,5-tetrahydro-1H-benzo[*d*]azepin-3-ium chloride on magnesium oxide as solid supporter

Into a flask equipped with magnetic stir bar was placed (*R*)-8-chloro-1-methyl-2,3,4,5-tetrahydro-1H-benzo[*d*]azepin-3-ium chloride (400 mg) which was dissolved in dichloromethane (10 mL). NanoActive® magnesium oxide (1 g) was added and the suspension was stirred at r.t. for 15 minutes. Solid was filtered off, washed with dichloromethane (2 x 5 mL) and dried under vacuum to obtain adsorbate (1.20 g; 17 % loading). Adsorbate was characterized with DSC and XPRD analysis where only amorphous material was detected within detection limits. The filtrate was concentrated for recovery of the lorcaserin.

**Example 9:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was dissolved in dichloromethane (8 mL). Syloid® 72 FP (1.2 g) was added in

portions and the suspension was stirred at r.t. for 30 minutes. Solvent was then slowly evaporated under reduced pressure and dried under vacuum to obtain dry adsorbate (1.42 g; 16 % loading). Adsorbate was characterized with DSC and XPRD analysis where only amorphous material was detected within detection limits.

**Example 10:** Adsorption of (*R*)-8-chloro-1-methyl-2,3,4,5-tetrahydro-1H-benzo[*d*]azepin-3-ium chloride on silica (Syloid® 244 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was dissolved in dichloromethane (10 mL). Syloid® 244 FP (1.2 g) was added in portions and the suspension was stirred at r.t. for 30 minutes. Solvent was then slowly evaporated under reduced pressure and dried under vacuum to obtain dry adsorbate (1.45 g; 83 % adsorption; 17 % loading). Material was characterized with DSC and XPRD where only amorphous material was detected within detection limits.

**Example 11:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was dissolved in ethanol (10 mL). Syloid® 72 FP (1 g) was added in portions and the suspension was stirred at r.t. for 30 minutes. Solvent was then slowly evaporated under reduced pressure and dried under vacuum to obtain dry adsorbate (1.25 g; 20 % loading). Material was characterized with DSC and routine XPRD analysis where only amorphous material was detected within detection limits.

**Example 12:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (200 mg) which was dissolved in methanol (8 mL). Syloid® 72 FP (800 mg) was added in portions and the suspension was stirred at r.t. for 30 minutes. Solvent was then slowly evaporated under reduced pressure and dried under vacuum to obtain dry adsorbate (945 mg). Material was characterized with DSC and routine XPRD analysis where only amorphous material was detected within detection limits. Stress stability of obtained dry material according to reference sample (lorcaserin hydrochloride crystalline form) was also tested and no sensitivity on temperature effect (60 °C), moisture effect (25-75% relative humidity) and oxidative stress (oxygen atmosphere) was detected. Assay of lorcaserin hydrochloride in adsorbate form did not change under stress conditions.

**Example 13:** Adsorption of (*R*)-8-chloro-1-methyl-2,3,4,5-tetrahydro-1H-benzo[*d*]azepin-3-ium chloride on silica (Syloid® 244 FP) as solid supporter

Into a flask equipped with magnetic stir bar was placed (*R*)-8-chloro-1-methyl-2,3,4,5-tetrahydro-1H-benzo[*d*]azepin-3-ium chloride (200 mg) which was dissolved in ethanol (10 mL). Syloid® 244 FP (800 mg) was added in portions and the suspension was stirred at r.t. for 30 minutes. Solvent was then slowly evaporated under reduced pressure and dried under vacuum to obtain dry adsorbate (995 mg; 20 % loading). Material was characterized with DSC and routine XPRD where only amorphous material was detected within detection limits.

**Example 14:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was suspended in 2-propanol (25 mL) and slowly heated between 40-45 °C to get clear solution. Syloid® 72 FP (1 g) was added in portions and the suspension was stirred at r.t. for 30 minutes. Solvent was then slowly evaporated under reduced pressure and dried under vacuum to obtain dry adsorbate (1.29 g). Material was characterized with DSC and XRD where only amorphous material was detected within detection limits.

**Example 15:** Adsorption of (*R*)-8-chloro-1-methyl-2,3,4,5-tetrahydro-1H-benzo[*d*]azepin-3-ium chloride on silica (Syloid® 72 FP) as solid supporter

Into a flask equipped with magnetic stir bar was placed (*R*)-8-chloro-1-methyl-2,3,4,5-tetrahydro-1H-benzo[*d*]azepin-3-ium chloride (300 mg) which was suspended in acetonitrile (30 mL) and slowly heated between 50-55 °C to get clear solution. Syloid® 72 FP (1 g) was added in portions and the suspension was stirred at r.t. for 30 minutes. Solvent

was then slowly evaporated under reduced pressure and dried under vacuum to obtain dry adsorbate (1.29 g). Material was characterized with DSC and XRD where only amorphous material was detected within detection limits.

**Example 16:** Direct one-pot preparation of adsorbate of (*R*)-8-chloro-1-methyl-2,3,4,5-tetrahydro-1H-benzo[*d*]azepin-3-ium chloride on silica (Syloid® 72 FP) solid supporter in alcoholic media

Into a flask equipped with magnetic stir bar was placed liquid (*R*)-8-chloro-1-methyl-2,3,4,5-tetrahydro-1H-benzo[*d*]azepine free base (293 mg) which was dissolved in ethanol or methanol (15 mL). Afterwards solution of hydrochloric acid in ethanol on methanol (1.44 ml of 1.25 M HCl) was slowly added and solution was stirred for 20 minutes. Syloid® 72 FP (1.05 g) was added in two portions and the suspension was stirred at r.t. for 30 minutes. Solvent was then slowly evaporated under reduced pressure and dried under vacuum to obtain dry adsorbate (1.32 g). Material was characterized with DSC and routine XRD where only amorphous material was detected within detection limits.

**Example 17:** Direct one-pot preparation of adsorbate of (*R*)-8-chloro-1-methyl-2,3,4,5-tetrahydro-1H-benzo[*d*]azepin-3-ium chloride on silica (Syloid® 72 FP) solid supporter in chlorinated solvents

Into a flask equipped with magnetic stir bar was placed liquid (*R*)-8-chloro-1-methyl-2,3,4,5-tetrahydro-1H-benzo[*d*]azepine free base (195 mg) which was dissolved in dichloromethane (15 mL). Afterwards aqueous solution of hydrochloric acid (1.25 ml of 1 M HCl) was slowly added and obtained two phase system was vigorously stirred for 30 minutes. Phases were separated and organic phase dried under $Mg_2SO_4$ which was then filtered off. Afterwards Syloid® 72 FP (960 mg) was added in two portions into solution and the suspension was stirred at r.t. for 30 minutes. Solvent was then slowly evaporated under reduced pressure and dried under vacuum to obtain dry adsorbate (1.32 g). Material was characterized with DSC and routine XRD where only amorphous material was detected within detection limits.

Notably, the routine evaluation of the above examples 1 to 17 revealed that within the detection limits the lorcaserin is confirmed to be fully adsorbed in an amorphous state to the inorganic adsorbent.

**Comparative Example 1:** Preparation of lorcaserin hydrochloride by fast solvent evaporation

To a 100 mL-round bottom flask were added lorcaserin free base (1.92 g, 9.81 mmol), 25 mL of dichloromethane and solution of hydrogen chloride (15.00 mmol). The mixture was stirred at room temperature for 5 min and the solvents were evaporated in rotary evaporator. The solid residue was dissolved in 25 mL of dichloromethane and treated with a solution of hydrogen chloride (15.00 mmol). The mixture was stirred at room temperature for 5 min and the solvents were fast evaporated in rotary evaporator (700 to 20 mbar, 40 °C), yielding crystalline lorcaserin hydrochloride (Forms II and III) as a light salmon powder (2.22 g, 9.56 mmol, 97 % yield, figure 1).

**Comparative Example 2:** Lyophilization of crystalline lorcaserin hydrochloride

100 g of crystalline lorcaserin hydrochloride, prepared according to Example 1, was dissolved in 6.0 mL of distilled water and 0.7 mL of ethanol. The homogeneous solution was fast frozen in a bath of liquid nitrogen followed by lyophilization at -40 °C and 0.2-0.4 mbar over 20 hours, again yielding crystalline lorcaserin hydrochloride.

## Claims

1. Amorphous lorcaserin hydrochloride ((*R*)-8-chloro-1-methyl-2,3,4,5-tetrahydro-1H-benzo[d]azepine hydrochloride) of formula 1

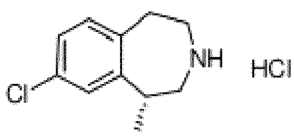

(formula 1)

stabilized by at least one pharmaceutically acceptable inorganic adsorbent.

2. Amorphous lorcaserin hydrochloride according to claim 1, wherein the at least one pharmaceutically acceptable inorganic adsorbent is selected from MgO and $SiO_2$, which is preferably selected from silica gel and fumed silica.

3. Amorphous lorcaserin hydrochloride according to claim 1 or 2, wherein the at least one pharmaceutically acceptable inorganic adsorbent is a particulate having a surface area of at least 150 $m^2/g$, preferably of at least 200 $m^2/g$.

4. Amorphous lorcaserin hydrochloride according to any of claims 1 to 3, wherein the silica gel and fumed silica is selected from Syloid®, preferably Syloid® 72 FP or Syloid® 244 FP, and Aerosil®, preferably Aerosil® 200, Aerosil® 300 and Aerosil® 380.

5. Amorphous lorcaserin hydrochloride according to any of claims 1 to 3, wherein the at least one pharmaceutically acceptable inorganic adsorbent is MgO selected from NanoActive® MgO.

6. Amorphous lorcaserin hydrochloride according to any of claims 1 to 5, wherein the loading of amorphous lorcaserin hydrochloride on the adsorbent is 12% or more, preferably 20% or more, more preferably 30% or more, still more preferably 40% or more.

7. Amorphous lorcaserin hydrochloride according to any of claims 1 to 6, wherein the loading of amorphous lorcaserin hydrochloride on the adsorbent is 65% or less, preferably 60% or less, more preferably 55% or less.

8. Process for the preparation of amorphous lorcaserin hydrochloride stabilized by at least one pharmaceutically acceptable inorganic adsorbent, the method comprising the steps (i) to (iv):

(i) dissolving lorcaserin hydrochloride in a protic solvent, an aprotic polar solvent, or a mixture thereof, or dissolving lorcaserin in said protic solvent, aprotic polar solvent, or mixture thereof upon *in situ* generation of lorcaserin hydrochloride;
(ii) adding at least one pharmaceutically acceptable inorganic adsorbent to the solution obtained in step (i);
(iii) mixing the dispersion obtained in step (ii); and
(iv) removing the solvent;

wherein the at least one pharmaceutically acceptable inorganic adsorbent may alternatively be dispersed in the protic solvent, aprotic polar solvent, or mixture thereof, before dissolving the lorcaserin or lorcaserin hydrochloride.

9. Process according to claim 8, wherein the protic solvent in step (i) is a C1 to C4 alcohol, preferably methanol, ethanol or 2-propanol, and/or
wherein the aprotic polar solvent in step (i) is a halogenated C1 to C4 hydrocarbon, preferably dichloromethane, or acetonitrile.

10. Process according to claims 8 or 9, wherein the step (i) is carried out at a temperature of 10 to 60 °C, preferably at 18 to 30 °C, more preferably 20 to 25 °C (r.t.), wherein if the protic solvent in step (i) is 2-propanol or the aprotic solvent in step (i) is acetonitrile a preferred temperature is 40-60 °C.

11. Process according to any of claims 8 to 10, wherein the at least one pharmaceutically acceptable inorganic adsorbent is defined according to any of claims 2 to 5.

12. Process according to any of claims 8 to 11, wherein the solvent is removed in the step (iv) by filtration, or
wherein the solvent is removed in the step (iv) by evaporation, preferably under reduced pressure.

13. A pharmaceutical composition comprising stabilized amorphous lorcaserin hydrochloride ((*R*)-8-chloro-1-methyl-2,3,4,5-tetrahydro-1H-benzo[d]azepine hydrochloride) according to any of claims 1 to 7, which optionally further comprises at least one pharmaceutically acceptable ingredient.

14. The pharmaceutical composition according to claim 13 for use as a medicament, preferably for use in the treatment of obesity.

15. The pharmaceutical composition according to claim 14 or 15, which is formulated for oral application, preferably in the form of tablets, capsules, pills or lozenges, and/or
wherein the pharmaceutical composition preferably comprises 5 to 100 mg, more preferably 7 to 20 mg, amorphous lorcaserin hydrochloride per dosage form.

Figure 1

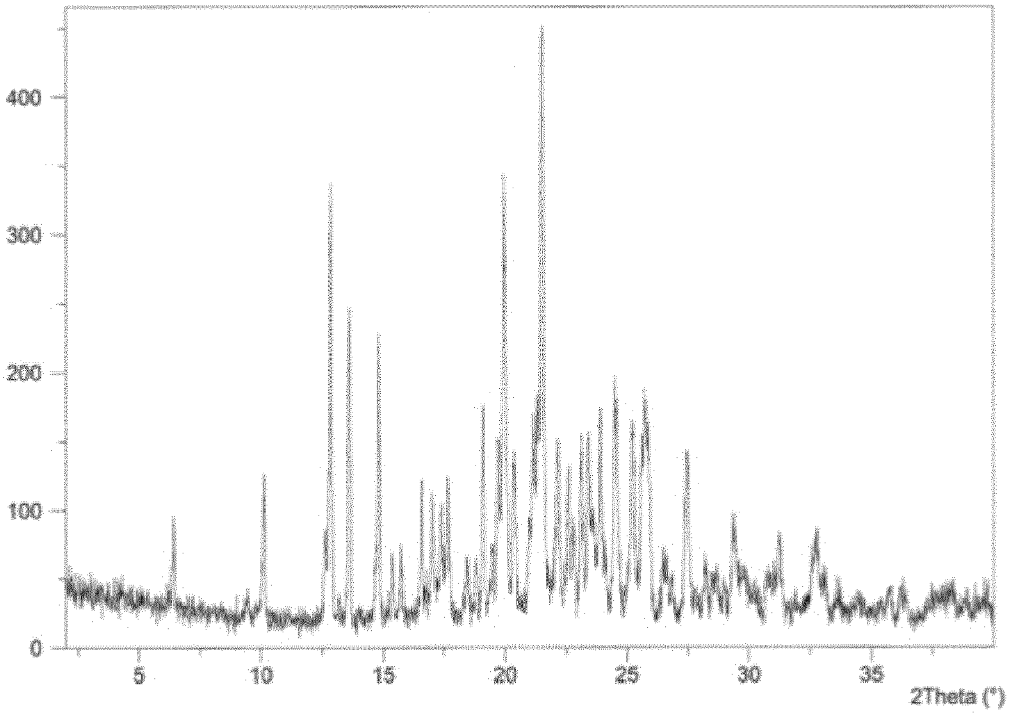

Europäisches Patentamt
European Patent Office
Office européen des brevets

# EUROPEAN SEARCH REPORT

Application Number
EP 13 19 1659

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2006/069363 A2 (ARENA PHARM INC [US]; AGARWAL RAJESH KUMAR [US]; BETTS III WILLIAM L [) 29 June 2006 (2006-06-29) * Page 3, lines 34-35; page 4, table 1; sentence bridging pages 10-11; pages 12-15, section "Pharmaceutical Formulations"; claims 1-15; figure 12. * | 1-15 | INV. C07D223/16 A61K31/55 A61P3/04 |
| Y,D | WO 2011/153206 A1 (ARENA PHARM INC [US]; BLACKBURN ANTHONY C [US]; SHIFRINA ANNA [US]) 8 December 2011 (2011-12-08) * Page 5, lines 7-15; page 14, line 9 to page 15, line 3; claims 1-18. * | 1-15 | |
| Y | WO 2012/030951 A1 (ARENA PHARM INC [US]; BLACKBURN ANTHONY C [US]; STIRN SCOTT [US]; SHAN) 8 March 2012 (2012-03-08) * Title; claims 24-26; page 5, lines 32-37; pages 86-93, example 11, step C of methods 1-3, i.e. preparation of lorcaserin HCl hemihydrate form III. * | 1-15 | TECHNICAL FIELDS SEARCHED (IPC) |
| Y | WO 2012/030927 A2 (ARENA PHARM INC [US]; SHAO ZEZHI JESSE [US]; BLACKBURN ANTHONY C [US];) 8 March 2012 (2012-03-08) * Title; page 4, line 20 to page 5, line 13; page 157, line 13 et seq. "Preparation of hydrochloride hemihydrate form III"; pages 158-159, example 5: Immediate Release Tablets; page 159 et seq., example 6; claims 6. * | 1-15 | C07D A61K A61P |
| | -/-- | | |

The present search report has been drawn up for all claims

1

| Place of search | Date of completion of the search | Examiner |
|---|---|---|
| Munich | 21 February 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 19 1659

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | Stephens: "NDA 22-529: Belviq (lorcaserin hydrochloride) tablets, Chemistry Review(s)", U.S. FDA CDER , 7 August 2012 (2012-08-07), pages 1-34, XP055103046, Retrieved from the Internet: URL:http://www.accessdata.fda.gov/drugsatfda_docs/nda/2012/022529Orig1s000ChemR.pdf [retrieved on 2014-02-19] * BNS DESC pages 7-8 of 26, section 2A "Description of Drug Product and Drug Substance"; BNS page 29, section "Drug Substance". * ----- | 1-15 | |
| A | RxList, Inc.: "Belviq (Lorcaserin Hydrochloride) Drug Description", , 5 July 2012 (2012-07-05), XP055103022, Retrieved from the Internet: URL:http://www.rxlist.com/script/main/rxlist.asp?articlekey=159981&pf=3&page=1 [retrieved on 2014-02-19] ----- | 1-15 | TECHNICAL FIELDS SEARCHED (IPC) |
| | -/-- | | |

1

The present search report has been drawn up for all claims

| Place of search | Date of completion of the search | Examiner |
|---|---|---|
| Munich | 21 February 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 19 1659

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| Y | HEIKKILÄ ET AL.: "Evaluation of Mesoporous TCPSi, MCM-41, SBA-15, and TUD-1 Materials as API Carriers for Oral Drug Delivery", DRUG DELIVERY,, vol. 14, 1 January 2007 (2007-01-01), pages 337-347, XP008147597, ISSN: 1071-7544, DOI: 10.1080/10717540601098823 * Abstract; page 338, left column, paragraph 2; page 342, left column, last paragraph and figure 5; page 346, section "Conclusion", in particular last paragraph. * ----- | 1-15 | |
| Y | AMBROGI ET AL.: "Amorphous carbamazepine stabilization by the mesoporous silicate SBA-15", MICROPOROUS AND MESOPOROUS MATERIALS, vol. 177, 19 April 2013 (2013-04-19), pages 1-7, XP028576349, ISSN: 1387-1811, DOI: 10.1016/J.MICROMESO.2013.04.008 * Title; abstract; page 1, right column, last paragraph to page 2, left column, end of paragraph 2. * ----- | 1-15 | TECHNICAL FIELDS SEARCHED (IPC) |
| Y | GODEC ET AL.: "Vitrification from solution in restricted space: Formation and stabilization of amorphous nifedipine in a nanoporous silica xerogel carrier", INTERNATIONAL JOURNAL OF PHARMACEUTICS, vol. 343, no. 1-2, 30 August 2007 (2007-08-30), pages 131-140, XP022223259, ISSN: 0378-5173, DOI: 10.1016/J.IJPHARM.2007.05.022 * Title, abstract; page 139, section "Conclusions". * ----- | 1-15 | |

The present search report has been drawn up for all claims

1

| Place of search | Date of completion of the search | Examiner |
|---|---|---|
| Munich | 21 February 2014 | Weisbrod, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 13 19 1659

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2014

| Patent document cited in search report | Publication date | Patent family member(s) | Publication date |
|---|---|---|---|
| WO 2006069363 A2 | 29-06-2006 | AT 442359 T | 15-09-2009 |
| | | AU 2005318959 A1 | 29-06-2006 |
| | | BR PI0519726 A2 | 10-03-2009 |
| | | CA 2589988 A1 | 29-06-2006 |
| | | CN 101084193 A | 05-12-2007 |
| | | CN 102321023 A | 18-01-2012 |
| | | CU 20070138 A7 | 22-01-2010 |
| | | DK 1838677 T3 | 11-01-2010 |
| | | EA 200701358 A1 | 28-12-2007 |
| | | EA 201200954 A1 | 30-05-2013 |
| | | EP 1838677 A2 | 03-10-2007 |
| | | EP 2149562 A1 | 03-02-2010 |
| | | EP 2327698 A1 | 01-06-2011 |
| | | ES 2332009 T3 | 22-01-2010 |
| | | HK 1102812 A1 | 31-12-2009 |
| | | HR P20090640 T1 | 31-01-2010 |
| | | IL 183844 A | 28-11-2013 |
| | | JP 5270167 B2 | 21-08-2013 |
| | | JP 2008524262 A | 10-07-2008 |
| | | JP 2012153727 A | 16-08-2012 |
| | | KR 20070098870 A | 05-10-2007 |
| | | MA 29147 B1 | 02-01-2008 |
| | | NI 200700160 A | 21-04-2008 |
| | | NZ 555981 A | 28-01-2011 |
| | | NZ 589756 A | 25-05-2012 |
| | | PT 1838677 E | 16-11-2009 |
| | | SI 1838677 T1 | 29-01-2010 |
| | | US 2010004223 A1 | 07-01-2010 |
| | | US 2012264743 A1 | 18-10-2012 |
| | | US 2014051684 A1 | 20-02-2014 |
| | | WO 2006069363 A2 | 29-06-2006 |
| | | ZA 200705123 A | 30-12-2009 |
| WO 2011153206 A1 | 08-12-2011 | KR 20130112848 A | 14-10-2013 |
| | | US 2013158013 A1 | 20-06-2013 |
| | | WO 2011153206 A1 | 08-12-2011 |
| WO 2012030951 A1 | 08-03-2012 | AU 2011296027 A1 | 04-04-2013 |
| | | CA 2808900 A1 | 08-03-2012 |
| | | CN 103189358 A | 03-07-2013 |
| | | EP 2611781 A1 | 10-07-2013 |
| | | JP 2013536858 A | 26-09-2013 |
| | | KR 20130138768 A | 19-12-2013 |
| | | SG 188363 A1 | 30-04-2013 |
| | | WO 2012030951 A1 | 08-03-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 19 1659

This annex lists the patent family members relating to the patent documents cited in the above-mentioned European search report. The members are as contained in the European Patent Office EDP file on
The European Patent Office is in no way liable for these particulars which are merely given for the purpose of information.

21-02-2014

| Patent document cited in search report | Publication date | Patent family member(s) | Publication date |
|---|---|---|---|
| WO 2012030927 A2 | 08-03-2012 | AU 2011296003 A1 | 04-04-2013 |
| | | CA 2808912 A1 | 08-03-2012 |
| | | CN 103189053 A | 03-07-2013 |
| | | EP 2611427 A2 | 10-07-2013 |
| | | JP 2013536857 A | 26-09-2013 |
| | | KR 20130137622 A | 17-12-2013 |
| | | SG 188362 A1 | 30-04-2013 |
| | | US 2013315994 A1 | 28-11-2013 |
| | | WO 2012030927 A2 | 08-03-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03086306 A1
- WO 2005019179 A2
- WO 2006069363 A2
- WO 2011153206 A1


### Non-patent literature cited in the description


- The Handbook of Pharmaceutical Excipients. American Pharmaceuticals Association, 2003
- Remington: the Science and Practice of Pharmacy. Lippincott Williams & Wilkins, 2000